# EUROPÄISCHE PATENTSCHRIFT

(11) **EP 1 216 985 B1**
(45) Veröffentlichungstag und Bekanntmachung des Hinweises auf die Patenterteilung: **14.02.2007**
(21) Anmeldenummer: 01128595.4
(22) Anmeldetag: 30.11.2001
(51) Int. Cl.: C07C 209/48

(54) **Verfahren zur Herstellung von 3-Aminomethyl-3,5,5-Trimethylcyclohexylamin**
Process for the preparation of 3-aminomethyl-3,5,5,-trimethylcyclohexylamine
Procédé pour la préparation de la 3-aminométhyl-3,5,5-triméthylcyclohexylamine

(30) Priorität: 23.12.2000 DE 10065030
(43) Veröffentlichungstag der Anmeldung: 26.06.2002
(73) Patentinhaber: Degussa AG, 40474 Düsseldorf (DE)
(72) Erfinder: Ostgard, Dr. Daniel, 63801 Kleinostheim (DE); Berweiler, Monika, 63477 Maintal (DE); Röder, Stefan, 36391 Sinntal (DE); Sauer, Jörg, Dr., Mobile, Alabama 36695 (US); Jaeger, Dr. Bernd, 64297 Darmstadt (DE); Finke, Dr. Norbert, 45739 Oerlinghausen-Erkenschwick (DE); Lettmann, Christian, 45259 Essen (DE)

(56) Entgegenhaltungen:
- EP-A- 0 880 996
- EP-A- 1 343 749
- DE-A- 2 301 139
- DE-A- 19 933 450
- DE-C- 19 540 191
- US-A- 5 554 573

## Beschreibung

Die Erfindung richtet sich auf ein verbessertes Verfahren zur Herstellung von 3-Aminomethyl-3,5,5- trimethylcyclohexylamin, nachfolgend auch Isophorondiamin oder abgekürzt IPDA genannt, aus 3-Cyano-3,5,5-trimethylcyclohexanon, nachfolgend auch Isophoronnitril oder abgekürzt IPN genannt durch aminierende Hydrierung in Gegenwart eines geformten Hydrierkatalysators auf Basis von Kobalt nach Raney. Bevorzugt umfaßt die Erfindung eine erste Stufe zur mindestens teilweisen Überführung von Isophoronnitril in Isophoronnitrilimin und eine zweite Stufe zur aminierenden Hydrierung des Reaktionsgemisches in Gegenwart eines Festbett-Hydrierkatalysators auf Basis von Kobalt nach Raney. Das Verfahren erlaubt die Herstellung von Isophorondiamin in gleich hohen oder höheren Ausbeuten bei gleichzeitigem Einsatz von deutlich geringeren Katalysatormengen als bei den bisher bekannten Methoden.

Isophorondiamin wird als Ausgangsprodukt zur Herstellung von Isophorondiisocyanat, einer Isocyanatkomponente für Polyurethansysteme, als Aminkomponente für Polyamide und als Härter für Epoxidharze verwendet. Isophorondiamin wird in vielen Fällen aus Isophoronnitril hergestellt, wobei in Gegenwart von Ammoniak, Wasserstoff und Hydrierkatalysatoren die Carbonylgruppe in eine Aminogruppe und die Nitrilgruppe in eine Aminomethylgruppe übergeführt werden. Das Ausgangsprodukt Isophoronnitril läßt sich in bekannter Weise durch Anlagerung von Cyanwasserstoff an Isophoron erhalten (siehe zum Beispiel DE-OS 39 42 371).

Raney-Katalysatoren auf Kobaltbasis sind aufgrund ihrer guten katalytischen Eigenschaften bei der Synthese von Isophorondiamin aus Isophoronnitril beziehungsweise Isophoronnitrilimin und ihrer im Vergleich zu Trägerkatalysatoren wesentlich leichteren Herstellung oftmals bevorzugt.

Raney-Katalysatoren die auch als aktivierte Metallkatalysatoren bezeichnet werden, bestehen aus einer Legierung aus mindestens einem katalytisch aktiven Metall und mindestens einem mit Alkalien auslaugbaren Metall. Für die in Alkalien lösliche Legierungskomponente wird vorwiegend Aluminium eingesetzt, es sind aber auch andere Metalle wie beispielsweise Zink und Silizium anwendbar. Durch Zugabe von Alkali zu der Legierung wird die auslaugbare Komponente herausgelöst, wodurch der Katalysator aktiviert wird.

Pulverkatalysatoren haben den Nachteil, daß sie nur im Batch-Verfahren eingesetzt werden können und nach der katalytischen Umsetzung aufwendig von den Reaktionsmedien abgetrennt werden müssen. Unter anderem aus diesem Grund ist es bevorzugt, die Herstellung von Isophorondiamin in kontinuierlichen Prozessen durchzuführen. Für diesen Zweck sind Festbettkatalysatoren notwendig, die neben einer guten katalytischen Aktivität auch eine ausreichend gute Festigkeit für den kontinuierlichen Betrieb aufweisen müssen.

In der Patentschrift DE 195 40 191 wird ein zweistufiges Verfahren zur Herstellung von Isophorondiamin beschrieben. In diesem Prozeß wird zunächst Isophoronnitril bei Anwesenheit oder Abwesenheit eines Iminierungskatalysators mit Ammoniak in das entsprechende Imin umgewandelt und das erhaltene Produktgemisch unter Zugabe von Wasserstoff zu Isophorondiamin hydriert. Als Hydrierkatalysator dient ein geformter Raney-Katalysator auf Basis von Kobalt. Der Katalysator enthält neben der Katalysatorlegierung aus Kobalt und Aluminium noch weiteres metallisches Kobalt, das als Binder für die nötige Stabilität des Formkörpers sorgt. Der Nachteil dieses Verfahrens liegt darin, daß das als Binder zugesetzte Kobalt nur wenig katalytisch aktiv ist, wodurch die Aktivität des Katalysators im Vergleich zu binderfreien Katalysatoren vermindert wird. Dadurch ist der Bedarf an Katalysator beziehungsweise an dem Metall Kobalt relativ hoch. Hohe Investitionskosten für das Kobalt sowie für die Auslegung der Reaktoren, beispielsweise bedingt durch das hohe Gewicht der Katalysatoren, sind die Folge.

Dieser Nachteil wird bei der Herstellung von Isophorondiamin vermieden, wie sie in dem Dokument EP 0 880 996 beschrieben wird. Zur Hydrierung wird ein geformter Kobaltkatalysator vom Raney-Typ verwendet, der vor der Aktivierung durch Auslaugen des Aluminiums ausschließlich aus einer Kobalt-Aluminium-Legierung besteht. Dieser Katalysator hat den Vorteil gegenüber den in dem Dokument DE 19540191 angewendeten Katalysator, daß er eine deutlich geringere Schüttdichte von nur 1,2 kg/l aufweist. Trotz der geringeren Schüttdichte führt bei gleicher Katalysatormasse die Hydrierung mit dem ausschließlich aus der Katalysatorlegierung bestehenden Katalysator zu leicht höheren Ausbeuten. Der Nachteil der in EP 0 880 996 beschriebenen Methode liegt darin, daß der verwendete Katalysator aber immer noch recht hohe Schüttdichten aufweist, relativ zu dem erfindungsgemäßen Katalysator.

In dem Dokument DE 199 33 450.1 werden Metallkatalysatoren beschrieben, die in Form von Hohlkörpern, bevorzugt in Form von Hohlkugeln vorliegen. Diese Katalysatoren besitzen eine niedrige Schüttdichte von 0,3 bis 1,3 g/ml. Neben den Katalysatoren wird außerdem deren Anwendung in Hydrierreaktionen beansprucht. In den Beispielen werden Aktivitätstests für die Hydrierung von Nitrobenzol zu Anilin aufgeführt, in denen bei Verwendung der hohlkugelförmigen Katalysatoren der Wasserstoffverbrauch und damit die Aktivität des Katalysators pro Gramm Katalysator deutlich höher ist als bei Verwendung eines Vergleichskatalysators. Die Herstellung von Isophorondiamin unter Anwendung der beschriebenen Katalysatoren wird als solche aber nicht genannt.

Die WO 02/051791 beschreibt ein Verfahren zur Herstellung von Aminen durch katalytische Hydrierung von Nitrilen oder Iminen mit Wasserstoff enthaltenden Gasen in Gegenwart eines geformten Hydrierkatalysators nach Raney, wobei der Raney-Katalysator in der Form von Hohlkörpern vorliegt.

Aufgabe der vorliegenden Erfindung ist daher, ein Verfahren zur Herstellung von Isophorondiamin aus Isophoronnitril zu entwickeln, bei dem die aminierende Hydrierung mit einem Festbett-Hydrierkatalysator nach Raney durchgeführt wird, der bei einer wesentlich geringeren Schüttdichte als vergleichbare Katalysatoren aber eine gleiche oder bessere Hydrieraktivität aufweist. Ein weiteres Ziel der Erfindung ist, bei einem Einsatz von weniger Katalysatormaterial im Vergleich zu bekannten Verfahren gleiche oder bessere Umsatzraten von Isophoronnitril beziehungsweise Isophoronnitrilimin zu erreichen.

Die zugrundeliegende Erfindung hat überraschenderweise gezeigt, daß bei der Herstellung von Isophorondiamin aus Isophoronnitrilimin oder Isophoronnitril durch aminierende Hydrierung mit Hilfe der in dem Dokument DE 199 33 450.1 beschriebenen hohlkörperförmigen Katalysatoren (bevorzugt Kobaltkatalysatoren) vom Raney-Typ deutlich höhere Umsatzraten pro Masseneinheit Katalysator zu erzielen sind als mit vergleichbaren Katalysatoren. Diese Beobachtung ist insofern überraschend, als daß man nicht zwangsläufig davon ausgehen kann, daß der hohlkörperförmige (Kobalt)Katalysator die erforderlichen Aktivitäten im speziellen Fall der Hydrierung von Isophoronnitril beziehungsweise Isophoronnitrilimin erreicht.

Gegenstand der Erfindung ist ein Verfahren zur Herstellung von Isophorondiamin aus Isophoronnitril, Isophoronnitrilimin oder Isophoronnitril und Isophoronnitrilimin enthaltenden Gemischen durch aminierende Hydrierung in Gegenwart von mindestens Ammoniak und Wasserstoff, wobei man einen geformten Raney-Katalysator auf Basis von Kobalt verwendet, dadurch gekennzeichnet, daß der Raney-Katalysator in der Form von Hohlkörpern vorliegt.

Dieses Verfahren hat den Vorteil, daß Isophorondiamin mit deutlich weniger Katalysatormaterial aber gleichen oder besseren Umsatzraten herzustellen ist, als es nach dem Stand der Technik bisher möglich ist.

Der dieser Erfindung zugrunde liegende Vorteil wird durch die Verwendung von Raney-Katalysatoren auf Kobaltbasis in Form von Hohlkörpern erreicht. Die Herstellung der in dem erfindungsgemäßen Verfahren angewendeten Katalysatoren kann entsprechend der in DE 199 33 450.1 beschriebenen Methode durchgeführt werden. Nach dieser Methode wird beispielsweise eine Mischung eines Kobaltlegierungspulvers mit einem auslaugbaren Metall, bevorzugt Aluminium, einem organischen Binder und gegebenenfalls Wasser sowie Promotoren auf Kugeln, die aus einem thermisch entfernbaren Material bestehen, aufgetragen.

Bevorzugt können Polystyrolschaumkugeln verwendet werden. Das Auftragen der die Metallegierung enthaltenden Mischung auf die Polymerkugeln kann bevorzugt in einem Wirbelbett durchgeführt werden. Als organische Binder können bevorzugt 0,0 bis 10 Gew.-% Polyvinylalkohol und/oder 0,0 bis 3 Gew.-% Glycerin eingesetzt werden. Die beschichteten Polymerschaumkugeln werden anschließend oberhalb von 300 °C, bevorzugt bei Temperaturen in einem Bereich zwischen 450 °C und 1300 °C calziniert, um den Polymerschaum thermisch zu entfernen und das Metall zu sintern. Dadurch erhalten die Hohlkörper eine stabilere Form. Nach der Calzinierung werden die hohlkörperförmigen Katalysatoren durch Behandeln mit basischen Lösungen, bevorzugt Alkali- oder Erdalkalihydroxide in Wasser, noch bevorzugter wäßrige Natronlauge aktiviert. Die so erhaltenen Katalysatoren besitzen Schüttdichten zwischen 0,2 bis 2,0 kg/l, bevorzugt 0,3 und 1.3 kg/l.

Erfindungsgemäß besitzen die in dem Verfahren angewendeten Katalysatoren die Form von Hohlkörpern. In einer bevorzugten Ausführungsform liegen die Raneykatalysatoren als Hohlkugeln vor. Hohlkugeln sind üblicherweise leicht herzustellen und besitzen eine hohe Bruchfestigkeit.

Die erfindungsgemäß angewendeten, hohlkörperförmigen Katalysatoren enthalten nach der Calzinierung und vor der Aktivierung bevorzugt keine Binder mehr. Es ist jedoch auch möglich, daß noch ein anorganischer Binder enthalten ist.

Die Schüttdichte der verwendeten Raney-Katalysatoren kann 0,3 g/ml bis 1,3 g/ml betragen.

Die verwendeten Katalysatorkörper können einen Durchmesser von 0,5 bis 20 mm aufweisen. Sie können eine Schalendicke von 0,1 bis 7,0 mm besitzen.

Die Kobaltlegierung der erfindungsgemäß verwendeten Katalysatoren ist bevorzugt zu 20 bis 80 Gew.-% aus Kobalt und zu 20 bis 80 Gew.-% aus einem mit Alkalien auslaugbaren Metall, bevorzugt Aluminium, zusammengesetzt. Als Kobaltlegierung kann eine schnell oder eine langsam abgekühlte Kobaltlegierung verwendet werden.

Unter schneller Abkühlung wird beispielsweise eine Abkühlung mit einer Rate von 10 bis 10⁵ K/s verstanden. Kühlmedien können verschiedene Gase oder Flüssigkeiten wie zum Beispiel Wasser sein. Unter langsamer Abkühlung versteht man Methoden mit kleineren Abkühlraten.

In dem erfindungsgemäßen Verfahren können mit anderen Metallen dotierte hohlkörperförmige Kobaltkatalysatoren verwendet werden. Die Dotierungsmetalle werden oftmals auch als Promotoren bezeichnet. Das Dotieren von Raney-Katalysatoren wird beispielsweise in den Dokumenten US 4,153,578, DE 21 01 856, DE 21 00 373 oder DE 20 53 799 beschrieben. Bevorzugte Metalle zum Dotieren sind Elemente der Gruppen 1A, 2A, 2B, 3B bis 7B, 8, 1B, 2B und/oder 3A des Periodensystems sowie Germanium, Zinn, Blei, Antimon und/oder Wismuth. Besonders bevorzugt sind Chrom, Mangan, Eisen, Vanadium, Tantal, Titan, Wolfram, Molybdän, Rhenium und/oder Metalle der Platingruppe. Der Anteil an Promotoren im Katalysator kann 0,0001 bis 50 Gew.-%, bevorzugt 0,001-20 Gew.-% betragen. Die Promotoren können bereits als Legierungsbestandteil enthalten sein, oder erst zu einem späteren Zeitpunkt, insbesondere nach der Aktivierung zugegeben werden.

In dem erfindungsgemäßen Verfahren werden bevorzugt hohlkörperförmigen Katalysatoren mit einem Durchmesser von 0,5 bis 20 mm und einer Schalendicke von 0,05 bis 7 mm verwendet. Die Katalysatorschalen können undurchlässig sein, oder eine Porosität von 0% bis zu 80% und höher aufweisen.

Die in dem erfindungsgemäßen Verfahren angewendeten hohlkörperförmigen Katalysatoren können aus einer oder mehreren Schichten bestehen. Haben die Katalysatorkörper mehrere Schichten, werden die Körper bei der Herstellung zwischen den einzelnen Beschichtungsschritten getrocknet. Dieses wird bevorzugt im Wirbelbett bei Temperaturen von 60 bis 150 °C durchgeführt.

Während des erfindungsgemäßen Verfahrens werden die hohlkörperförmigen Kobaltkatalysatoren vom Raney-Typ in der aktivierten Form eingesetzt. Das in den nicht aktivierten Katalysatorkörpern vorhandene auslaugbare Metall kann im aktivierten Zustand ganz oder nur teilweise mit Alkalien herausgelaugt worden sein.

In dem erfindungsgemäßen Verfahren zur Herstellung von Isophorondiamin wird der beschriebene hohlkörperförmige Kobaltkatalysator für den Schritt der aminierenden Hydrierung von Isophoronnitril beziehungsweise von Isophoronnitrilimin eingesetzt. Dieser Prozeß kann batchweise oder kontinuierlich durchgeführt werden.

Es ist möglich, das erfindungsgemäße Verfahren in einer Stufe oder in mehreren Stufen durchzuführen. Wird das Verfahren in einer Stufe durchgeführt, wird Isophoronnitril direkt in Anwesenheit von Ammoniak, Wasserstoff, dem hohlkörperförmigen Kobaltkatalysator und gegebenenfalls weiteren Zusätzen und in An- oder Abwesenheit von organischen Lösungsmitteln aminierend hydriert. Der Begriff "in mehreren Stufen" bedeutet, daß zunächst in einem separaten Reaktor oder Reaktorabschnitt Isophoronnitril ganz oder teilweise in Isophoronnitrilimin umgewandelt wird, und dieses Isophoronnitrilimin als Reinsubstanz oder in einem Gemisch mit anderen Komponenten unter Anwesenheit von Ammoniak aminierend hydriert wird. Die erfindungswesentliche Anwendung des hohlkörperförmigen Kobaltkatalysators ist bei der aminierenden Hydrierung von Bedeutung.

Die Reaktionsbedingungen, d. h. Druck und Temperatur, sowie das Verhältnis von IPN, NH₃, Wasserstoff und gegebenenfalls des organischen Lösungsmittels sind bei einstufiger und zweistufiger Fahrweise identisch und entsprechen den Bedingungen der Hydrierstufe bei der zweistufigen Fahrweise.

Eine bevorzugte Ausführungsform des erfindungsgemäßen Verfahrens zur Herstellung von Isophorondiamin ist ein zweistufiger Prozeß: In der ersten Stufe wird zumindest ein Teil des eingesetzten Isophoronnitrils bei An- oder Abwesenheit eines Iminierungskatalysators und/oder von Lösungsmitteln in Isophoronnitrilimin umgewandelt. Das Verhältnis von Isophoronnitrilimin zu Isophoronnitril sollte nach dem Iminierung größer 1, bevorzugt größer als 4 und noch bevorzugter größer als 9 sein. In der zweiten Stufe wird das Reaktionsprodukt der ersten Stufe, so wie es anfällt oder nach einer Weiterbehandlung, unter Anwesenheit von mindestens Ammoniak und Wasserstoff und in Anwesenheit oder Abwesenheit eines organischen Lösungsmittels bei einer Temperatur von 20 bis 150 °C , bevorzugt 60 bis 150 °C und einem Druck von 0,3 bis 50 MPa, bevorzugt 5 bis 10 MPa aminierend hydriert, gegebenenfalls mit Hilfe von hohlkörperförmigen Kobaltkatalysatoren hydriert.

Die Iminierung kann in Anwesenheit oder Abwesenheit eines Iminierungskatalysators durchgeführt werden. Sofern die Iminierung in Abwesenheit eines Iminierungskatalysators durchgeführt wird, sind bei einer Reaktionstemperatur im Bereich zwischen 10 und etwa 60 °C mehrere Stunden erforderlich, um den erwünschten Iminierungsgrad zu erreichen. Bei höheren Temperaturen besteht die Gefahr einer stärkeren Nebenproduktbildung, was die Reinheit des Endprodukts Isophorondiamin stark beeinflussen würde. Weitere Aufarbeitungs- und Reinigungsschritte wären notwendig.

Um die Gleichgewichtseinstellung der Iminierungsreaktion zu beschleunigen, ist es zweckmäßig, einen Iminierungskatalysator zu verwenden. Hierzu können die nach dem Stand der Technik bekannten Iminierungskatalysatoren verwendet werden. Geeignete Katalysatoren sind beispielsweise anorganische oder organische Ionentauscher (siehe EP 0 042 119), trägergebundene Heteropolysäuren (siehe DE 44 26 472), acide Metalloxide, insbesondere Aluminiumoxid und Titandioxid (Anatas) (siehe EP 0 449 089), Sulfonsäuregruppen enthaltende Organopolysiloxane (DE 196 27 265.3) und saure Zeolithe. Bei Verwendung eines Iminierungskatalysators kann die Reaktionstemperatur zwischen 10 und 150 °C, vorzugsweise zwischen 30 und 130 °C und insbesondere zwischen 40 und 120 °C liegen. Die Iminierungsreaktion wird bevorzugt bei Drücken im Bereich von Atmosphärendruck bis 50 MPa, bevorzugt bei Drücken im Bereich bis 30 MPa durchgeführt. Besonders bevorzugt ist der Druck, bei dem auch die anschließende Hydrierung durchgeführt wird.

Obwohl die Iminierung von Isophoronnitril mit flüssigem Ammoniak in Abwesenheit eines weiteren Lösungsmittels möglich ist, kann es vorteilhaft sein, zusätzlich ein Lösungsmittel aus der Reihe eines Alkohols mit 1 bis 4 C-Atomen, vorzugsweise eines einwertigen primären Alkohols und insbesondere Methanol, oder eines Ethers wie Tetrahydrofuran, MTBE (= Methyl-Tertiärbutylether) oder Dioxan zu verwenden. Vorzugsweise wird dem Iminierungsreaktor ein Isophoronnitril, flüssiges Ammoniak und Methanol enthaltendes Gemisch zugeführt. Das Gemisch enthält zweckmäßigerweise 10 bis 40 Gew.-%, vorzugsweise 10 bis 30 Gew.-% Isophoronnitril und 10 bis 40 Gew.-%, vorzugsweise 20 bis 40 Gew.-% Ammoniak. Es ist vorteilhaft, Isophoronnitril, Ammoniak und das Lösungsmittel in einem solchen Verhältnis miteinander zu mischen, daß eine im wesentlichen homogene Mischung resultiert. Im Prinzip können die zuvor genannten Grenzwerte auch unter- oder überschritten werden, sofern hierbei eine im wesentlichen homogene Lösung entsteht. Durch die Verwendung des organischen Lösungsmittels ist es möglich, die Iminierungsreaktion bei niedrigeren Drücken durchzuführen, als dies bei Abwesenheit des Lösungsmittels möglich wäre. Bei Anwesenheit des Lösungsmittels liegen die bevorzugten Drücke im Bereich von 2 bis 10 MPa.

Bei der Iminierung in Gegenwart eines Iminierungskatalysators kann der Katalysator in Form eines Suspensionskatalysators oder eines Festbettkatalysators zur Anwendung gelangen. Vorteilhaft ist die Verwendung eines Festbettkatalysators, da sich hierbei aufwendige Schritte zur Abtrennung des Reaktionsgemisches vom Katalysator erübrigen. Bei der Iminierung von Isophoronnitril in Gegenwart eines Festbettkatalysators wird dieser in Form üblicher Katalysatorformlinge wie Stangenpreßlinge, Pellets und Tabletten als Schüttung in einem Festbettreaktor eingesetzt. Der Iminierungskatalysator kann in einem eigenen Reaktor angeordnet sein. Es ist jedoch auch möglich, den Iminierungskatalysator in einem Reaktor anzuordnen, der sowohl eine Schüttung des Iminierungskatalysators, als auch eine Schüttung des für die aminierende Hydrierung eingesetzten Katalysators enthält. Je nachdem, ob der Reaktor als Rieselbett- oder Blasenreaktor betrieben wird, befindet sich die Schüttung des Iminierungskatalysators oberhalb (Rieselbettreaktor) oder unterhalb (Blasenreaktor) der Schüttung des Hydrierkatalysators. Es ist auch möglich, einen einzigen Reaktor zu verwenden, der sowohl eine Schüttung des Hydrierkatalysators als auch eine Schüttung des Iminierungskatalysators enthält. In diesem Fall laufen in einem Reaktor, jedoch in zwei getrennten Reaktorabschnitten beide Stufen der Isophorondiaminsynthese ab.

Vorzugsweise wird ein solcher Reaktor in Form eines Rieselbettreaktors betrieben. Hierbei wird das Gemisch aus Isophoronnitril, Ammoniak und Lösungsmittel, insbesondere Alkohol und/oder Ether, am Reaktorkopf aufgegeben. In diesen Fällen strömt zweckmäßigerweise gleichzeitig Wasserstoff für die aminierende Hydrierung von oben in den Reaktor.

Außer den zuvor genannten Bestandteilen des der Iminierungsstufe zuzuführenden Gemischs kann dieses zusätzlich höher oder tiefer als Isophorondiamin siedende Fraktionen aus der destillativen Aufarbeitung des aus dem Rieselbettreaktor abgezogenen Reaktionsgemisches enthalten. Derartige Fraktionen können außer Resten an Isophorondiamin auch solche Nebenprodukte enthalten, aus welchen sich unter den Reaktionsbedingungen erneut Isophorondiamin bildet. Durch Rückführung derartiger Fraktionen in das einzusetzende Gemisch läßt sich die Ausbeute an Isophorondiamin deutlich steigern. Besonders vorteilhaft ist es, die oberhalb von Isophorondiamin siedende Fraktion, welche außer Resten an Isophorondiamin 3,3,5-Trimethyl-6-imino-7-azabicyclo[3,2,1] octan als Hauptprodukt enthält, zusammen mit dem Gemisch aus Isophoronnitril, Ammoniak und Lösungsmittel, bevorzugt Methanol und/oder MTBE, dem Rieselbettreaktor zuzuführen. Durch die Rückführung der das vorgenannte Nebenprodukt -eine bicyclische Verbindung mit Amidinstruktur - enthaltenden Fraktion ist es möglich, die Ausbeute an Isophorondiamin nennenswert zu steigern und damit die Wirtschaftlichkeit des Verfahrens zu erhöhen. Die das bicyclische Amidin enthaltende Fraktion kann, falls dies erwünscht ist, auch unmittelbar dem der zweiten Stufe zuzuführenden Reaktionsgemisch zugesetzt werden.

Die entscheidenden Verbesserung des erfindungsgemäßen Verfahrens besteht in der Verwendung des bereits beschriebenen hohlkörperförmigen Kobaltkatalysators bei der aminierenden Hydrierung. In dem bevorzugten zweistufigen Prozeß wird ein Isophoronnitrilimin enthaltendes Gemisch mit Hilfe des hohlkörperförmigen Kobaltkatalysators hydriert. Das genannte Gemisch kann unmittelbar jenes sein, wie es bei der Iminierung von Isophoronnitril mit Ammoniak in Gegenwart oder Abwesenheit eines organischen Lösungsmittels, wie beispielsweise Methanol und/oder MTBE, in An- oder Abwesenheit eines Iminierungskatalysators erhalten wird oder wie es aus einem derartigen Reaktionsgemisch nach Zugabe oder Abdestillieren von Lösungsmitteln und/oder eines Teils des Ammoniaks erhältlich ist. Dabei ist aus denselben Gründen, wie sie bereits bei der Iminierung aufgeführt wurden, die kontinuierlich betriebene Hydrierung in einem Festbettreaktor bevorzugt, es ist jedoch auch eine Batch-Fahrweise oder die Durchführung in einer Rührkesselkaskade möglich. Wie bereits bei der Iminierungsreaktion abgehandelt, kann der Reaktor sowohl als Rieselbettreaktor als auch als Blasensäule betrieben werden, wobei jedoch die Rieselbettfahrweise bevorzugt ist.

Es ist bevorzugt, daß bei dem aminierenden Hydrierungsschritt in dem das Isophoronnitrilimin enthaltende Gemisch ein organisches Lösungsmittel, bevorzugt ein aliphatischer C₁ bis C₄-Alkohol, insbesondere Methanol, oder ein Ether, insbesondere MTBE oder Tetrahydrofuran enthalten ist. In diesem Fall ist es möglich, die aminierende Hydrierung bei niedrigeren Drücken durchzuführen, als bei Abwesenheit eines solchen Lösungsmittels. Sofern bei der im ersten Schritt durchgeführten Iminierung noch kein organisches Lösungsmittel im Reaktionsgemisch enthalten war, kann man das organische Lösungsmittel, bevorzugt Methanol oder MTBE, auch zu dem aminierend zu hydrierenden Reaktionsgemisch zugeben. Es ist jedoch auch möglich, ohne Zugabe eines Lösungsmittels zu arbeiten.

Es ist auch möglich, für die Hydrierung mehrere Rieselbettreaktoren hintereinander zu schalten, wobei das aus dem ersten Reaktor austretende Reaktionsgemisch am Kopf des zweiten Reaktors wieder aufgegeben wird. Mit dieser Methode ist es möglich, den Hydrierungsschritt weiter zu unterteilen. Der Aufbau und Fahrweise solcher Reaktoren ist aus dem Stand der Technik bekannt.

Der für die Hydrierung erforderliche Wasserstoff kann dem Reaktor entweder im Überschuß, beispielsweise mit bis zu 10000 Moläquivalenten, zugeführt werden oder in einer solchen Menge, daß kein Wasserstoff aus dem Reaktor ausgetragen und recycliert werden muß. Bei Anwesenheit eines Lösungsmittel im Reaktionsgemisch wird vorzugsweise Wasserstoff nicht im Überschuß zugeführt, um den technischen Aufwand zur Abtrennung dieses Überschusses, zur Kondensation des in ihm enthaltenen Ammoniaks und Lösungsmittels sowie zur Kompression des gereinigten Wasserstoffs zu vermeiden. Ist kein Lösungsmittel im Reaktionsgemisch enthalten, kann die aminierende Hydrierung mit einem Wasserstoffüberschuß von 1 bis 30 Moläquivalenten durchgeführt werden. Wird das erfindungsgemäße Verfahren in einem kontinuierlichen Prozeß durchgeführt, kann der Wasserstoff im Gleich- oder im Gegenstrom zugeführt werden.

Ist bei der aminierenden Hydrierung gemäß der bevorzugten Ausführungsform ein Lösungsmittel, beispielsweise MTBE oder Methanol, im Reaktionsgemisch enthalten, kann die aminierende Hydrierung, also der zweite Reaktionsschritt, üblicherweise bei einer Temperatur im Bereich von 20 bis 150 °C, vorzugsweise 90 bis 130 °C und bei einem Druck im Bereich von 0,3 bis 10 MPa, vorzugsweise bei 5 bis 8 MPa und insbesondere bei 8 MPa durchgeführt werden. Durch die genannten mäßigen Betriebsdrücke, welche bei der Verwendung der bevorzugten Gemische aus Isophoronnitril, Ammoniak, Wasserstoff und Lösungsmittel unter den angegebenen Temperaturbedingungen möglich sind, wird das Investitionsvolumen gemindert und damit die Wirtschaftlichkeit gegenüber Verfahren, welche einen hohen Betriebsdruck erfordern, erhöht. Unter dem angegebenen Druck wird der Gesamtdruck verstanden, der sich aus den Partialdrücken von Ammoniak, Wasserstoff, C₁- bis C₄- Alkohol sowie den übrigen Bestandteilen des Reaktionsgemisches zusammensetzt. Es ist jedoch auch möglich, die aminierende Hydrierung in anderen Temperatur- oder Druckbereichen wie beispielsweise zwischen 150 und 250 °C oder bei Drücken bis zu 50 MPa durchzuführen, beispielsweise wenn kein organisches Lösungsmittel im Reaktionsgemisch enthalten ist.

Bei der Hydrierung von Isophoronnitril beziehungsweise Isophoronnitrilimin können zwei unterschiedliche Stereosisomere gebildet werde. Es kann bevorzugt sein, durch die Wahl eines Temperaturprogramms im Hydrierschritt das Isomerenverhältnis zu beeinflussen. Es ist beispielsweise möglich ein Isophoronnitril oder Isophoronnitrilimin enthaltendes Gemisch zunächst bei einer Temperatur im Bereich zwischen 20 und 90 °C, und in einem sich daran anschließenden Abschnitt bei einer Temperatur im Bereich zwischen 90 und 150 °C zu hydrieren, wobei der Temperaturunterschied der beiden Hydrierabschnitte mindestens 30 °C betragen sollte. Ein solches Temperaturprogramm bei der Hydrierung kann beispielsweise dadurch erreicht werden, daß die Hydrierstufe in zwei Unterabschnitte mit voneinander getrennten Reaktoren aufgeteilt wird. Auf diese Art und Weise ist es möglich, die Selektivität zu Gunsten des cis-Isomers zu verschieben.

Die aminierende Hydrierung wird in Anwesenheit von Ammoniak durchgeführt. Pro Mol Nitril beziehungsweise Imin werden üblicherweise 2 oder mehr Mol Ammoniak, meistens 5 bis 500 Mol Ammoniak eingesetzt. Zweckmäßigerweise kann man das Ammoniakangebot, das bei der vorgelagerten Herstellung von Isophoronnitrilimin eingestellt wurde, wählen. Ammoniak dient außer zur Iminierung teilweise oder - n Abwesenheit eines anderen Lösungsmittels wie Methanol oder Tetrahydrofuran - auch ganz als Lösungsmittel.

Das erforderliche Volumen an Festbettkatalysator für die Hydrierstufe richtet sich nach dem vom Betriebsdruck, der Temperatur und Katalysatoraktivität abhängigen LHSV-Wert (liquid hour space velocity) der eingehalten werden muß, um einen möglichst quantitativen Umsatz des Isophoronnitrilimin und Isophoronnitril enthaltenden Gemisches zu erzielen. Üblicherweise beträgt der LHSV-Wert bei der Verwendung der bevorzugten Gemische aus Isophoronnitril, Ammoniak, Wasserstoff und Lösungsmittel mindestens 0,5 h⁻¹ und liegt vorzugsweise im Bereich von I h⁻¹ bis 4 h⁻¹ und noch bevorzugter bei ungefähr 2 h⁻¹ bis 3 h⁻¹.

Das den Hydrierreaktor verlassende Reaktionsgemisch wird in an sich bekannter Weise aufgearbeitet. Diese Aufarbeitung umfaßt üblicherweise Abtrennen des Ammoniaks, der Lösungsmittel oder Gemische aus Ammoniak und Lösungsmittel, falls Lösungsmittel vorhanden sind, sowie Isolation des IPDA.

Unabhängig davon, ob das erfindungsgemäße Verfahren zur Herstellung von Isophorondiamin in einer bevorzugten Ausführungsform durchführt wird oder nicht, können bei der Umsetzung eines Gemisches aus Isophoronnitril, Ammoniak, Wasserstoff und gegebenenfalls einem Lösungsmittel noch eine oder mehrere Hydroxidbasen zugegeben werden.

Eine häufige Nebenreaktion bei der Hydrierung von Nitrilen ist die Bildung von sekundären Aminen. Dieses wird durch einen Austausch der Iminofunktion verursacht, bei dem bereits gebildetes primäres Amin durch Verdrängung von Ammoniak aus der bei der Hydrierung durchlaufenen Zwischenstufe des Imins ein neues, n-alkyliertes Imin bildet, das anschließend weiter zum sekundären Amin hydriert wird. Insbesondere diese Nebenreaktion wird durch Zugabe von Basen vermindert oder nahezu vollständig unterdrückt. Dies gilt auch für die intramolekulare Iminbildung, also die Bildung von 2-Aza-4,6,6-trimethylbicyclo[3,2,1]-Octan.

Die Zugabe kann vor der Iminierung des Isophoronnitrils oder auch erst nach der vollständigen oder teilweisen Iminierung und vor der Hydrierung zugegeben werden. Die Zugabe von Hydroxidbasen kann die Ausbeute an Isophorondiamin steigern und/oder die Reinheit des Isophorondiamins erhöhen. Geeignete Hydroxidbasen sind beispielsweise Alkalihydroxide oder Erdalkalihydroxide. Besonders bevorzugte Hydroxidbasen sind quaternäre Ammoniumhydroxide. Geeignete Ammoniumhydroxide sind solche der allgemeinen Formel (R¹R²R³R⁴)⁺ OH⁻, wobei R¹ bis R⁴ gleich oder unterschiedlich sein können und für aliphatische, cycloaliphatische oder aromatische Reste stehen. Beispiele sind Tetramethyl-, Tetraethyl-, Tetra-n- propyl- und Tetra-n-butylammoniumhydroxid. Geeignete Konzentrationen sind 0,01 bis 100 mmol, bevorzugt 0,05 bis 20 mmol eines Tetraalkylammoniumhydroxids pro Mol des Isophoronnitrils.

Es ist auch möglich, bei der erfindungsgemäßen Hydrierung von Isophoronnitril oder Isophoronnitrilimin mit einem hohlkörperförmigen Kobaltkatalysator einen oder mehrere Co-Katalysatoren zu verwenden. Geeignete Co-Katalysatoren sind Salze von Kobalt, Nickel, Lanthan, Cer oder Yttrium, bevorzugt Salze von Kobalt und Nickel. Eine bevorzugte Menge an Co-Katalysator liegt bei 0,01 bis 0,5 Mol, bevorzugt 0,05 bis 0,2 Mol Co-Katalysator pro Mol Kobaltkatalysator. Der oder die Co-Katalysatoren können in Form wasserfreier oder Kristallwasser enthaltender Salze, in Pulverform, als Lösung oder als Suspension dem Kobaltkatalysator oder der Reaktionsmischung zugegeben werden.

Es ist auch möglich, jedoch nicht bevorzugt, Isophorondiamin in einem einstufigen Prozeß aus Isophoronnnitril durch aminierende Hydrierung mit einem hohlkörperförmigen Kobaltkatalysator nach Raney herzustellen. Bei diesem Prozeß wird Isophoronnitril in Anwesenheit von Ammoniak in situ in Isophoronnitrilimin umgewandelt, das dann weiter zu Isophorondiamin hydriert wird. Der einstufige Prozeß wird bevorzugt kontinuierlich in einem Festbettreaktor in der Art durchgeführt, daß der Reaktor als Rieselbettreaktor verwendet wird (siehe beispielsweise EP 0 659 734).

Das erfindungsgemäße Verfahren zur Herstellung von Isophorondiamin aus Isophoronnitril durch aminierende Hydrierung hat folgende Vorteile:
Der erfindungsgemäß verwendete hohlkörperförmige Kobaltkatalysator vom Raney-Typ besitzt eine deutlich geringere Schüttdichte als bisher verwendete Raney-Katalysatoren. Dadurch wird bei der Herstellung von Isophorondiamin wesentlich weniger Katalysatormaterial benötigt, als in den bisher bekannten Verfahren.

Trotz der deutlich geringeren Menge an Katalysatormaterial kann die Herstellung von Isophorondiamin mit hohen Umsatzraten, sehr guten Ausbeuten und sehr guten Raum-Zeit- Ausbeuten durchgeführt werden.

Da weniger Katalysatormaterial benötigt wird, ist der technische Aufwand für die bei der Herstellung von Isophorondiamin eingesetzten Reaktoren niedriger. Dieses ist beispielsweise durch das geringere Gewicht des eingesetzten Kobaltkatalysators begründet.

### Beispiele

### Anwendungsbeispiel:

Die Katalysatoren werden bei der Herstellung von 3-Aminomethyl-3,5,5-Trimethylcyclohexylamin (Isophorondiamin, IPDA) aus 3-Cyano-3,5,5-trimethylcyclohexanon (Isophoronnitril, IPN) in einem zweistufigen Verfahren auf ihre katalytische Wirksamkeit geprüft. Das Verfahren ist im wesentlichen in der DE 195 40 191 C1 beschrieben.

In der ersten Stufe wird dabei Isophoronnitril in Anwesenheit eines Iminierungskatalysators mit Ammoniak und unter Zugabe von Methanol zumindest teilweise in 3-Cyano-3,5,5-trimethylcyclohexamin überführt und in der zweiten Stufe an einem Hydrierungskatalysator bei einer Temperatur von 100 °C und einem Druck von 6 MPa mit Wasserstoff aminierend hydriert.

Jede Stufe der Herstellung von IPDA wird in Abweichung von der in der DE 195 40 191 C1 beschriebenen Vorgehensweise in separaten Reaktoren durchgeführt. Beide Reaktoren sind jedoch hintereinandergeschaltet. Sie werden durch getrennte Ölheizungen temperiert.

Das erste Reaktorrohr hat einen Innendurchmesser von 20 mm und eine Länge von 250 mm und ist mit 30 ml eines Sulfonatgruppen enthalenden Organopolysiloxanes (Körngröße 0,4 bis 1,4 mm; Schüttdichte 525 g/l) als Iminierungskatalysator befüllt (siehe DE Patentanmeldung 196 27 265.3).

Der Hydrierreaktor besitzt einen Innendurchmesser von 17 mm und eine Länge von 350 mm und wird bei jedem Versuch mit 150 ml des jeweils zu prüfenden Katalysators befüllt.

Die Temperatur des ersten Reaktors wird auf 35 °C und die Temperatur im zweiten Reaktor auf 100 °C eingestellt. Der Druck in beiden Reaktoren beträgt 6 MPa.

Die Einsatzlösung aus IPN (15 Gew.-%), Ammoniak (30 Gew.-%) und Methanol (55 Gew.-%) wird mit einem Massenstrom von 80 ml/h von unten durch das erste Reaktionsrohr gepumpt; das dabei erhaltene iminierte Reaktionsgemisch läuft von dort auf den zweiten Reaktor. Der Wasserstoff wird von oben in das zweite Reaktionsrohr mit einem Riesel-Volumenstrom von 36 l/h eingeleitet, der Reaktor also als Rieselbettreaktor betrieben. Die Produktflüssigkeit wird unter dem Reaktor in einem Abscheidegefäß aufgefangen.

Das aufgefangene Produktgemisch wird auf IPDA und entsprechende Nebenprodukte gaschromatographisch untersucht. Die Untersuchungsergebnisse sind in Tabelle 1 aufgeführt.

### Beispiel 1

Es wird eine Beschichtungslösung hergestellt, indem man 800 g einer Legierung aus 50 % Co / 50 % Al in 1.000 ml wäßriger Lösung mit einem Gehalt an 5 Gew.-% Polyvinylalkohol und 1,25 Gew.-% Glycerin suspendiert.

Diese Suspension wird sodann auf 2.000 ml Polystyrolkugeln im Bereich um 2 mm aufgesprüht, während diese in einem nach oben gerichteten Luftstrom suspendiert sind. Nach dem Beschichten der Polystyrolkugeln mit der zuvor genannten Lösung werden die Kugeln sodann in aufwärtsströmender Luft bei Temperaturen bis zu 80 °C getrocknet (höhere Temperaturen können auch angewendet werden).

Die Hälfte von diesen getrockneten, beschichteten Polystyrolkugeln wird mit einer Legierungslösung weiter beschichtet. Die Lösung für die zweite Schicht besteht aus 800 g einer Legierung aus 50 % Co / 50 % Al, die in 1.000 ml einer wäßrigen Lösung mit einem Gehalt an 5 Gew.-% Polyvinylalkohol und 1,25 Gew.-% Glycerin suspendiert ist. Diese Suspension wird sodann auf 1.000 ml der zuvor genannten, mit Co/Al vorbeschichteten und getrockneten Polystyrolkugeln aufgesprüht, während diese in einem nach oben gerichteten Luftstrom suspendiert sind. Die zweite Beschichtung kann auch mit einer anderen Legierung erfolgen, die andere Metalle enthält und/oder Teilchen mit verschiedenen Korngrößenverteilungen hat, so daß die entsprechende katalytische Hohlkugel besondere Eigenschaften, wie bimodale Metall- und/oder Porenverteilung wegen der besonders gestalteten Beschichtung, zeigen kann.

Nach dem Beschichten der Polystyrolkugeln mit den zuvor erwähnten Lösungen werden diese sodann in einem gesteuerten Stickstoff/Luftstrom auf 700 °C erwärmt, um das Polystyrol herauszubrennen und die Legierungsteilchen zusammenzusintern. Die Hohlkugeln werden sodann in einer 20 Gew.-%igen Natronlauge 1,5 Std. bei 80 °C aktiviert. Die erhaltenen aktivierten Hohlkugeln besitzen Durchmesser im Bereich um 3 mm, eine Manteldicke um 700 µm und eine Schüttdichte von 0,80 g/ml.

Wie aus der Entwicklung von Wasserstoffblasen visuell zu sehen ist, hat der Katalysator ein großes Reservoir aktiven Wasserstoffs.

Laut des zuvor erwähnten Anwendungsbeispiels hat die Prüfung der aktivierten Kobalthohlkugeln (Katalysator B1) für die Herstellung des IPDA eine IPDA-Ausbeute von 94,4 % und eine IPDA-Reinheit von 99,9 % nach der destillativen Aufarbeitung gezeigt.

### Vergleichsbeispiel 1:

Ein handelsüblicher Kobalt-Trägerkatalysator (Co auf einem Silikat) wird als Hydrierkatalysator, laut des zuvor erwähnten Anwendungsbeispiels für die Herstellung von IPDA getestet. Dieser Katalysator (VB1) hat nach der destillativen Aufarbeitung gemäß Beispiel 1 eine IPDA-Ausbeute von 90,2 % und eine IPDA-Reinheit von 99,75 % gezeigt.

**Tabelle 1**

| Katalysator | IPDA-Ausbeute | IPDA-Reinheit |
|---|---|---|
| B1 | 94,4 | 99,9 |
| VB1 | 90,2 | 99,75 |

## Patentansprüche

1. Verfahren zur Herstellung von Isophorondiamin aus Isophoronnitril, Isophoronnitrilimin oder Isophoronnitril und Isophoronnitrilimin enthaltenden Gemischen durch aminierende Hydrierung in Gegenwart von mindestens Ammoniak und Wasserstoff, wobei man einen geformten Raney-Katalysator auf Basis von Kobalt verwendet, **dadurch gekennzeichnet, daß** der Raney-Katalysator in der Form von Hohlkörpern vorliegt.

2. Verfahren nach Anspruch 1, **dadurch gekennzeichnet, daß** das Verfahren in zwei Stufen durchgeführt wird, umfassend eine erste Stufe, in der Isophoronnitril in Ab- oder Anwesenheit eines Iminierungskatalysators und/oder eines organischen Lösungsmittels mit Ammoniak zumindest teilweise in Isophoronnitrilimin überführt wird, und eine zweite Stufe, in der das Reaktionsgemisch der ersten Stufe in Ab- oder Anwesenheit eines organischen Lösungsmittels bei einer Temperatur im Bereich von 20 bis 150 °C und einem Druck im Bereich von 0,3 bis 50 MPa aminierend hydriert wird.

3. Verfahren nach Anspruch 1 oder 2, **dadurch gekennzeichnet, daß** der Raney-Katalysator in Form von Hohlkugeln vorliegt.

4. Verfahren nach Anspruch 1 oder 3, **dadurch gekennzeichnet, daß** die Schüttdichte der verwendeten Raney-Katalysatoren im Bereich von 0,3 g/ml bis 1,3 g/ml liegt.

5. Verfahren nach Anspruch 1 oder 4, **dadurch gekennzeichnet, daß** die verwendeten Katalysatorkörper einen Durchmesser im Bereich von 0,5 bis 20 mm besitzen.

6. Verfahren nach einem oder mehreren der Ansprüche 1 bis 5, **dadurch gekennzeichnet, daß** die verwendeten Katalysatorkörper eine Schalendicke im Bereich von 0,1 bis 7,0 mm besitzen.

7. Verfahren nach einem oder mehreren der Ansprüche 1 bis 6, **dadurch gekennzeichnet, daß** die im Verfahren verwendeten Katalysatorkörper keinen Binder enthalten.

8. Verfahren nach einem oder mehreren der Ansprüche 1 bis 6, **dadurch gekennzeichnet, daß** die im Verfahren verwendeten Katalysatorkörper einen anorganischen Binder enthalten.

9. Verfahren nach einem oder mehreren der Ansprüche 1 bis 8, **dadurch gekennzeichnet, daß** der verwendete Kobaltkatalysator mit Elementen aus den Gruppen 3B bis 7B, 8 und 1B des Periodensystems, insbesondere Chrom, Mangan, Eisen, Vanadium, Tantal, Titan, Wolfram, Molybdän, Rhenium und/oder Metalle der Platingruppe dotiert wird.

10. Verfahren nach einem oder mehreren der Ansprüche 1 bis 8, **dadurch gekennzeichnet, daß** der verwendete Kobaltkatalysator mit Elementen aus den Gruppen 1A, 2A, 2B und/oder 3A des Periodensystems und/oder Germanium, Zinn, Blei, Antimon und/oder Wismuth dotiert wird.

11. Verfahren nach einem oder mehreren der Ansprüche 2 bis 10, **dadurch gekennzeichnet, daß** die Iminierung des Isophoronnitrils in Gegenwart eines Iminierungskatalysators und/oder eines Alkohols und/oder Ethers durchgeführt wird.

12. Verfahren nach einem oder mehreren der Ansprüche 2 bis 11, **dadurch gekennzeichnet, daß** die aminierende Hydrierung in einem Festbett- oder Suspensions-Reaktor in kontinuierlichem Betrieb durchführt wird.

13. Verfahren nach Anspruch 12, **dadurch gekennzeichnet, daß** man die aminierende Hydrierung im Rieselbettverfahren durchführt, wobei das aminierend zu hydrierende Reaktionsgemisch im Reaktor oder in hintereinander geschalteten Reaktoren eine oder mehrere mit steigender Temperatur angeordnete Temperaturstufen durchläuft.

14. Verfahren nach einem oder mehreren der Ansprüche 2 bis 13, **dadurch gekennzeichnet, daß** bei der aminierenden Hydrierung ein Co-Katalysator aus der Reihe eines Kobalt- oder Nickelsalzes anwesend ist.

15. Verfahren nach einem oder mehreren der Ansprüche 2 bis 14, **dadurch gekennzeichnet, daß** bei der aminierenden Hydrierung Wasserstoff nicht im Überschuß zugegeben wird.

16. Verfahren nach einem oder mehreren der Ansprüche 2 bis 11, **dadurch gekennzeichnet, daß** man den gesamten Prozeß oder einzelne Stufen des Prozesses im Batch-Verfahren durchführt.

17. Verfahren nach einem oder mehreren der Ansprüche 1 bis 16, **dadurch gekennzeichnet, daß** bei der aminierenden Hydrierung ein basisches Material anwesend ist.

18. Verfahren nach einem der Ansprüche 1 oder 3, **dadurch gekennzeichnet, daß** das Verfahren in zwei Stufen durchgeführt wird, umfassend eine erste Stufe, in der Isophoronnitril in Ab- oder Anwesenheit eines Iminierungskatalysators und in Abwesenheit eines organischen Lösungsmittels mit Ammoniak zumindest teilweise in Isophoronnitrilimin überführt wird, und eine zweite Stufe, in der das Reaktionsgemisch der ersten Stufe in Abwesenheit eines organischen Lösungsmittels bei einer Temperatur im Bereich von 60 bis 150 °C und einem Druck im Bereich von 5 bis 50 MPa aminierend hydriert wird.

19. Verfahren nach Anspruch 17, **dadurch gekennzeichnet, dass** Alkalihydroxid, Erdalkalihydroxid oder Ammoniumhydroxid anwesend sind.

## Claims

1. Process for the preparation of isophorone diamine from isophorone nitrile, isophorone nitrilimine or mixtures containing isophorone nitrile and isophorone nitrilimine by hydrogenation through to amine in the presence of at least ammonia and hydrogen using a formed Raney catalyst based on cobalt **characterized in that** the Raney catalyst is in the form of hollow bodies.

2. Process according to Claim 1, **characterized in that** the process is carried out in two stages including a first stage in which isophorone nitrile is converted at least partially with ammonia into isophorone nitrilimine in the absence or in the presence of an imination catalyst and/or of an organic solvent, and a second stage in which the reaction mixture of the first stage is hydrogenated through to amine in the absence or in the presence of an organic solvent at a temperature within the range 20 to 150°C and at a pressure within the range 0.3 to 50 MPa.

3. Process according to Claim 1 or 2, **characterized in that** the Raney catalyst is in the form of hollow spheres.

4. Process according to Claim 1 or 3, **characterized in that** the Raney catalysts used have a bulk density within the range 0.3 g/ml to 1.3 g/ml.

5. Process according to Claim 1 or 4, **characterized in that** the catalyst bodies used have a diameter within the range 0.5 to 20 mm.

6. Process according to one or more of Claims 1 to 5, **characterized in that** the catalyst bodies used have a shell thickness within the range 0.1 to 7.0 mm.

7. Process according to one or more of Claims 1 to 6, **characterized in that** the catalyst bodies used in the process contain no binder.

8. Process according to one or more of Claims 1 to 6, **characterized in that** the catalyst bodies used in the process contain an inorganic binder.

9. Process according to one or more of Claims 1 to 8, **characterized in that** the cobalt catalyst used is doped with elements from Groups 3B to 7B, 8 and 1B of the Periodic Table, in particular chromium, manganese, iron, vanadium, tantalum, titanium, tungsten, molybdenum, rhenium and/or metals in the platinum group.

10. Process according to one or more of Claims 1 to 8, **characterized in that** the cobalt catalyst used is doped with elements from Groups 1A, 2A, 2B, and/or 3A of the Periodic Table and/or germanium, tin, lead, antimony and/or bismuth.

11. Process according to one or more of Claims 2 to 10, **characterized in that** the imination of isophorone nitrile is in the presence of an imination catalyst and/or an alcohol and/or ether.

12. Process according to one or more of Claims 2 to 11, **characterized in that** the hydrogenation through to amine is carried out in a fixed-bed reactor or suspension reactor in continuous operation.

13. Process according to Claim 12, **characterized in that** the hydrogenation through to amine is carried out in the trickle-bed process, where the reactor or reactors are connected in series and the reaction mixture which is to be hydrogenated through to amine runs through one or more temperature stages arranged with increasing temperatures.

14. Process according to one or more of Claims 2 to 13, **characterized in that** a co-catalyst from the series of a cobalt or nickel salt is present in the hydrogenation through to amine.

15. Process according to one or more of Claims 2 to 14, **characterized in that** hydrogen is not added in excess in the hydrogenation through to amine.

16. Process according to one or more of Claims 2 to 11, **characterized in that** the entire process or individual stages of the process are carried out in the batch process.

17. Process according to one or more of Claims 1 to 16, **characterized in that** a basic material is present in the hydrogenation through to amine.

18. Process according to one of Claims 1 or 3, **characterized in that** the process is carried out in two stages, including a first stage in which isophorone nitrile is converted at least partially with ammonia in the absence or in the presence of an imination catalyst and in the absence of an organic solvent into isophorone nitrilimine, and a second stage, in which the reaction mixture of the first stage is hydrogenated through to amine in the absence of an organic solvent at a temperature within the range 60 to 150°C and at a pressure within the range 5 to 50 MPa.

19. Process according to Claim 17, **characterized in that** alkali hydroxide, alkaline earth hydroxide or ammonium hydroxide are present.

## Revendications

1. Procédé pour la préparation d'isophoronediamine à partir de mélanges contenant de l'isophoronenitrile, de l'isophoronenitrilimine ou de l'isophoronenitrile et de l'isophoronenitrilimine par hydrogénation avec amination au moins en présence d'ammoniac et d'hydrogène, avec utilisation d'un catalyseur de Raney façonné à base de cobalt, **caractérisé en ce que** le catalyseur de Raney se trouve sous forme de corps creux.

2. Procédé selon la revendication 1, **caractérisé en ce que** le procédé est réalisé en deux étapes, comprenant une première étape, dans laquelle l'isophoronenitrile est transformé en l'absence ou en présence d'un catalyseur d'imination et/ou d'un solvant organique avec de l'ammoniac, au moins partiellement en isophoronenitrilimine et une deuxième étape, dans laquelle le mélange réactionnel de la première étape est hydrogéné avec amination en l'absence ou en présence d'un solvant organique à une température dans la plage de 20 à 150°C et à une pression dans la plage de 0,3 à 50 MPa.

3. Procédé selon la revendication 1 ou 2, **caractérisé en ce que** le catalyseur de Raney se trouve sous forme de billes creuses.

4. Procédé selon la revendication 1 ou 3, **caractérisé en ce que** la densité apparente des catalyseurs de Raney utilisés se trouve dans la plage de 0,3 g/ml à 1,3 g/ml.

5. Procédé selon la revendication 1 ou 4, **caractérisé en ce que** les corps de catalyseur utilisés présentent un diamètre dans la plage de 0,5 à 20 mm.

6. Procédé selon l'une ou plusieurs des revendications 1 à 5, **caractérisé en ce que** les corps de catalyseur utilisés présentent une épaisseur de coquille dans la plage de 0,1 à 7,0 mm.

7. Procédé selon l'une ou plusieurs des revendications 1 à 6, **caractérisé en ce que** les corps de catalyseur utilisés dans le procédé ne contiennent pas de liant.

8. Procédé selon l'une ou plusieurs des revendications 1 à 6, **caractérisé en ce que** les corps de catalyseur utilisés dans le procédé contiennent un liant inorganique.

9. Procédé selon l'une ou plusieurs des revendications 1 à 8, **caractérisé en ce que** le catalyseur à base de cobalt utilisé est dopé avec des éléments des groupes 3B à 7B, 8 et 1B du système périodique, en particulier le chrome, le manganèse, le fer, le vanadium, le tantale, le titane, le tungstène, le molybdène, le rhénium et/ou des métaux du groupe du platine.

10. Procédé selon l'une ou plusieurs des revendications 1 à 8, **caractérisé en ce que** le catalyseur à base de cobalt utilisé est dopé avec des éléments des groupes 1A, 2A, 2B et/ou 3A du système périodique et/ou avec du germanium, de l'étain, du plomb, de l'antimoine et/ou du bismuth.

11. Procédé selon l'une ou plusieurs des revendications 2 à 10, **caractérisé en ce que** l'imination de l'isophoronenitrile est réalisée en présence d'un catalyseur d'imination et/ou d'un alcool et/ou d'un éther.

12. Procédé selon l'une ou plusieurs des revendications 2 à 11, **caractérisé en ce que** l'hydrogénation avec amination est réalisée dans un réacteur à lit fixe ou à suspension dans un fonctionnement continu.

13. Procédé selon la revendication 12, **caractérisé en ce qu'**on réalise l'hydrogénation avec amination dans un procédé à lit à écoulement, le mélange réactionnel à hydrogéner avec amination passant, dans le réacteur ou dans des réacteurs disposés les uns derrière les autres, une ou plusieurs étapes de température disposées avec une température croissante.

14. Procédé selon l'une ou plusieurs des revendications 2 à 13, **caractérisé en ce que** lors de l'hydrogénation avec amination, un cocatalyseur de la série d'un sel de cobalt ou de nickel est présent.

15. Procédé selon l'une ou plusieurs des revendications 2 à 14, **caractérisé en ce que** l'hydrogène n'est pas ajouté en excès lors de l'hydrogénation avec amination.

16. Procédé selon l'une ou plusieurs des revendications 2 à 11, **caractérisé en ce qu'**on réalise l'ensemble du procédé ou les différentes étapes du procédé dans un procédé par lots.

17. Procédé selon l'une ou plusieurs des revendications 1 à 16, **caractérisé en ce qu'**un matériau basique est présent lors de l'hydrogénation avec amination.

18. Procédé selon l'une quelconque des revendications 1 ou 3, **caractérisé en ce que** le procédé est réalisé en deux étapes, comprenant une première étape, dans laquelle l'isophoronenitrile est transformé, en l'absence ou en présence d'un catalyseur d'imination et en l'absence d'un solvant organique avec de l'ammoniac, au moins partiellement en isophoronenitrilimine et une deuxième étape, dans laquelle le mélange réactionnel de la première étape est hydrogéné avec amination en l'absence d'un solvant organique à une température dans la plage de 60 à 150°C et à une pression dans la plage de 5 à 50 MPa.

19. Procédé selon la revendication 17, **caractérisé en ce que** de l'hydroxyde de métal alcalin, de l'hydroxyde de métal alcalino-terreux ou de l'hydroxyde d'ammonium sont présents.
